# EUROPEAN PATENT APPLICATION

(11) **EP 4 512 247 A1**
(43) Date of publication of application: **26.02.2025**
(21) Application number: 24192104.8
(22) Date of filing: 31.07.2024
(51) Int. Cl.: A01N 63/22, C12N 1/20, A23B 2/783

(54) **FERMENTED PH-INDEPENDENT SOLUTION SPOILAGE CONTROL IN FOOD SYSTEMS**

(30) Priority: 21.08.2023 US 202318236009
(71) Applicant: Third Wave Bioactives, LLC, Wauwatosa, WI 53226 (US)
(72) Inventor: Stafl, Sydney, Milwaukee, 53222 (US); Gebert, Shelly, Brookfield, 53045 (US); Hundt, Matthew, Brookfield, 53005 (US)
(74) Representative: Rommeswinkel, Marike

(57) **Abstract**

A fermentate and methods for producing a fermentate and for killing or inhibiting the growth of a contaminating microorganism on or within a food product using a GRAS strain of *Bacillus* that does not produce organic acids at a level effective to inhibit the growth of microorganisms but does contain metabolites that have antibacterial and antifungal activity at a broad range of pH including >5.5 is disclosed. A food product comprising a fermentate having a cellular mass component from a GRAS strain of *Bacillus* is also disclosed. The GRAS strain of *Bacillus* may be selected from a group consisting of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis,* and *Bacillus toyonensis.*

## Description

### FIELD

The present application generally relates to fermented products for use in preventing the spoilation of food.

### BACKGROUND

The Background and Summary are provided to introduce a foundation and selection of concepts that are further described below in the Detailed Description. The Background and Summary are not intended to identify key or essential features of the claimed subject matter, nor are they intended to be used as an aid in limiting the scope of the claimed subject matter.

The shelf life for foods can be prolonged in many ways. Improving product packaging can decrease the surface area of the food exposed, limiting the ability of aerobic microorganisms to grow. Modified atmosphere packaging can ensure the product has minimal to no oxygen exposure while properly sealed in its original packaging. Formula and ingredient-based solutions are also effective when the product quality allows for such interventions. A decrease in water activity or a_{w} (a measure of water freely available in the system for microbial metabolism) below 0.80 will prevent all but the most extreme osmophilic or xerophilic fungi species to proliferate, however this requires a reduction in moisture that is not feasible in many foods such as deli meats or baked goods. Similarly, an increase in salt will present a barrier to microbial growth but will affect the flavor and nutritional profile of the food.

Consumer demand for fewer and more recognizable ingredients has led ingredient suppliers to find creative ways to remove artificial ingredients and replace them with friendlier, more natural alternatives. Familiarity is one key aspect ingredient producers must consider when generating such solutions. Several ingredient manufacturers market and sell a variety of flavor and antimicrobial products based on fermentation. Today, these products involve culturing food-grade bacteria (Generally Regarded As Safe (GRAS)) on various substrates, such as sucrose, dextrose, whey, non-fat dry milk, wheat flour, and wheat starch. The resulting fermented products are labeled as "cultured wheat flour", "cultured skim milk", etc., and are subsequently added to foods to improve flavor and/or to provide improved shelf-life and food safety. These are often viewed as "label-friendly" (or, "clean label") alternatives to synthetic preservatives (such as potassium sorbate, sodium benzoate, calcium propionate, etc.).

While these ingredients make sense when used in fresh dairy products and baked goods, they also contain allergens and make little sense in other applications, such as soups, dips, dressings and meat products. Additionally, consumers may be less familiar with an ingredient like "dextrose" and might question why "cultured dextrose", for example, would be included as a component of a refrigerated soup. In addition to allergenicity, these "cultured" products also often have a flavor profile that -- while better than that of synthetic preservatives -- may not be entirely in line with the foods within which they're being utilized. Finally, some of the fermented technologies that exist today are not allowed in all food categories, limiting their use by default. Given these limitations, there is a need in the art for preservative products that are easily recognizable by consumers, lack allergens, and provide minimal, if not improved, sensory impact.

U.S. Patent No. 11,213,055 discloses fermentate compositions comprising reuterin (β-hydroxypropionaldehyde) that display antimicrobial activity against a variety of microorganisms in foods. The fermentate compositions may not only be active at acidic pHs, but also retain antimicrobial activity at elevated pHs including neutral pHs. In one embodiment, the fermentate is a *Lactobacillus reuteri* fermentate.

Su, Y., Liu, C., Fang, H. et al. explain in Bacillus subtilis: a universal cell factory for industry, agriculture, biomaterials and medicine, Microb Cell Fact 19, 173 (2020), that *Bacillus* fermentations have been widely studied and are already used across many industries ranging from agriculture to pharmaceuticals. In many of these cases, the *Bacillus* strain is genetically modified to produce a specific metabolite of interest.

Strains of *Bacillus amyloliquefaciens* have been identified in published findings as a beneficial antifungal endophytes in Zeng, Fan-Song et al. "Complete Genome Sequence of Bacillus amyloliquefaciens EA19, an Endophytic Bacterium with Biocontrol Potential Isolated from Erigeron annuus." Microbiology Resource Announcements Vol. 10,39 (2021): e0075321. doi:10.1128/MRA.00753-21. Additionally, *Bacillus amyloliquefaciens* has been shown to express antifungal activity in soil and root systems attributed to the production of lipopeptides linked with the formation of *in situ* biofilms in Xu, Zhihui et al. "Contribution of bacillomycin D in Bacillus amyloliquefaciens SQR9 to antifungal activity and biofilm formation." Applied and Environmental Microbiology Vol. 79,3 (2013): 808-15. doi:10.1128/AEM.02645-12.

### SUMMARY

This Summary is provided to introduce a selection of concepts that are further described below in the Detailed Description. This Summary is not intended to identify key or essential features of the claimed subject matter, nor is it intended to be used as an aid in limiting the scope of the claimed subject matter.

In one aspect of the present invention, fermentates selected from one of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis, Bacillus toyonensis,* or another GRAS species of *Bacillus* are provided. The fermentates may include a cellular mass component from a fermenting microorganism and a substrate providing a carbon source, including but not limited to sucrose, dextrose, lactose, starches, fruits, vegetables, whole grains, whey, milk or extracts thereof.

In another aspect, the present invention relates to methods for producing a fermentate. Such methods may include producing a fermentate by obtaining a substrate, adding a strain selected from one of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis, Bacillus toyonensis,* or another GRAS species of *Bacillus* to the substrate to produce a fermenting composition; and incubating the fermenting composition at a controlled temperature in a high oxygen environment to produce a fermentate. In one embodiment, the strain selected from one of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis, Bacillus toyonensis,* or another GRAS species of *Bacillus* that does not produce organic acids at a level effective to inhibit the growth of microorganisms. In another embodiment, the strain is selected from one of one of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis,* and *Bacillus toyonensis.* In certain embodiments the strain is of *Bacillus amyloliquefaciens* strain. In another embodiment, the fermentate exhibits antibacterial and antifungal activity. In yet another embodiment, the fermentate exhibits antibacterial and antifungal activity at a pH above 5.5.

In the methods described above, the strain of *Bacillus amyloliquefaciens* may be strain 723. In the methods described above, the controlled temperature may be between about 10° C and about 50° C. In the methods described above, the high oxygen environment is a liquid environment where the dissolved oxygen concentration in liquid is between 40% and 100%. In still other embodiments, the dissolved oxygen concentration in liquid is between 50% and 99%, and the liquid is aerated consistently by filtered atmospheric air and mechanical agitation between 50-500 RPM during incubation.

In other embodiments, a method for killing or inhibiting the growth of a contaminating microorganism on or within a food product is disclosed. The food product has a volume, and the method includes the steps of making or obtaining a fermentate comprising a cellular mass component from a strain selected from one of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis, Bacillus toyonensis,* or another GRAS species of *Bacillus* and a substrate; and applying an effective amount of the fermentate to the food product so as to kill or inhibit the growth of the contaminating microorganism on or within the food product. In certain embodiments the strain is selected from one of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis,* and *Bacillus toyonensis.* In one embodiment, the fermenting organism is a strain of *Bacillus amyloliquefaciens.*

In certain embodiments, the fermentate may be applied in a concentration between about 0.1% and about 5% of the food product volume. The food product may be selected from the group consisting of culinary items, bakery items, cereals, pasta, meats, dairy items, rice, fish, nuts, beverages, confections, pet food, fruits, and vegetables. The contaminating microorganism may be selected from the group consisting of a yeast species, a mold species, a gram-positive bacteria, and a gram negative bacteria, or any combination thereof. In certain embodiments, the strain of *Bacillus amyloliquefaciens* is strain 723. In certain embodiments, the strain of *Bacillus* does not produce organic acids at a level effective to inhibit the growth of microorganisms, and in other embodiments, the fermentate exhibits antibacterial and antifungal activity. In still other embodiments, the fermentate may exhibit antibacterial and antifungal activity at a pH above 5.5

In certain embodiments of the method, the substrate provides a carbon source for the fermenting organism and is preferably selected from the group consisting of: sucrose, dextrose, lactose, starches, fruits, vegetables, whole grains, whey, milk or extracts thereof. The fruits may be selected from any of apples, watermelons, oranges, pears, strawberries, grapes, plum, mangos, blueberries, papaya, apricots, mandarins, bananas, grapefruit, lemons, limes, pineapples, jackfruits, melons, coconuts, avocados, peaches, kiwi, blackcurrants, blackberries, cherries, figs, nectarines, passionfruit, quince, raspberries, tangerines, pomegranates, mulberries, starfruit, guava, pomelos, cranberries, cantaloupe, tamarinds, persimmons, or a combination thereof. The vegetables may be selected from beets, cassavas, parsnips, beans, legumes, lentils, peas, potatoes, sweet potatoes, corn, plantains, taro, pumpkins, squash, turnips, or a combination thereof. The whole grains may be selected from any of wheat, barley, rice, rye, or a combination thereof. The fermentate may be a concentrated liquid or a dry powder. The fermentate may be produced by any one of the methods described above.

In still other embodiments, a food product comprising a fermentate having a cellular mass component from a strain selected from one of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis, Bacillus toyonensis,* or another GRAS species of *Bacillus* is disclosed. The food product may have a pH above 5.5. The food product may be selected from the group consisting of culinary items, bakery items, cereals, pasta, meats, dairy items, rice, fish, nuts, beverages, confections, pet food, fruits, and vegetables. The food product may have a volume and include the fermentate in a concentration between about 0.1% and about 5% of the food product volume. In certain embodiments the strain is selected from one of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis,* and *Bacillus toyonensis.* In one embodiment, the fermenting organism is a strain of *Bacillus amyloliquefaciens.* In certain embodiments, the strain of *Bacillus amyloliquefaciens* is strain 723. The strain of *Bacillus* may be selected from a strain that does not produce organic acids at a level effective to inhibit the growth of microorganisms. The fermentate may exhibit antibacterial and antifungal activity.

The fermentate may be a concentrated liquid or a dry powder. In certain embodiments, the fermentate has the ability to inhibit the growth of a contaminating microorganism by 100% when diluted to less than 5% (w/v).

These and still other advantages of the invention will be apparent from the detailed description and drawings. Various other features, objects, and advantages will be made apparent from the following description taken together with the drawings. What follows is merely a description of some preferred embodiments of the present invention. To assess the full scope of the invention, the claims should be looked to as these preferred embodiments are not intended to be the only embodiments within the scope of the claims.

### BRIEF DESCRIPTION OF THE DRAWINGS

**FIG. 1** shows the growth of yeast in a 10% potato flake slurry at pH 6.0 treated with a fermentate of the present disclosure compared to an untreated control held at room temperature.
**FIG. 2** shows the growth of yeast in almond milk at pH 7.8 treated with a fermentate of the present disclosure compared to an untreated control held at refrigerated temperature.
**FIG. 3** shows the growth of yeast in chicken salad at pH 6.2 treated with a fermentate of the present invention plus 500ppm nisin compared to an untreated control, 500ppm nisin, and 0.1% Potassium sorbate plus 500ppm nisin.
**FIG. 4** shows the growth of lactic acid bacteria in chicken salad at pH 6.2 treated with a fermentate of the present invention plus 500ppm nisin compared to an untreated control, 500ppm nisin, and 0.1% Potassium sorbate plus 500ppm nisin.
**FIG. 5** demonstrates the growth of inoculated yeast population (Y-1545 and Y-866) in control and treated samples of chicken broth stored at 4C for 53 days.
**FIG. 6** demonstrates growth of inoculated yeast population (Y-1545 and Y-866) in control and treated samples of vegetable broth stored at 4C for 80 days.

### DETAILED DESCRIPTION

Any citations references are made herein are incorporated by reference herein in their entireties. In the event that there is an inconsistency between a definition of a term in the specification as compared to a definition of the term in a cited reference, the term should be interpreted based on the definition in the specification.

Here, in the non-limiting Examples, the present inventors have surprisingly discovered that culturing bacteria selected from one of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis, Bacillus toyonensis* or another GRAS species of *Bacillus* can produce effective antimicrobial and/or antifungal fermentates, including in high pH systems. The inventors envision that the disclosed fermentates would allow food manufacturers to use preservative products that have efficacious antimicrobial and/ or antifungal activity at a more neutral pH.

### Fermentate Compositions

In one aspect of the present invention, fermentates are provided. As used herein, a "fermentate" refers to a complex mixture produced by a controlled fermentation process. The fermentate may include a cellular mass component from a microorganism including, without limitation, fermentation end-products, metabolites, and/or unused substrates. The present application contemplates a fermentate produced by any one of the methods described herein.

The fermentates may include a cellular mass component from a fermenting microorganism and a substrate. In some embodiments, the fermentate may be made or produced by any one of the methods for producing a fermentate disclosed herein.

As used herein, the "cellular mass component" refers to the proteins, lipids (i.e., membranes), carbohydrates, metabolites, etc., or any subset of these substances from the fermenting microorganism. For example, as a fermenting microorganism grows it produces new cells that generally include additional cellular mass such as, without limitation, cell membranes, nucleic acids (i.e., DNA and/or RNA) internal subcellular structures, small molecules such as organic acids, or proteins (i.e., membrane-bound, secreted, and/or intracellular). The cellular mass component may include all of these substances or only some of these substances. For example, a fermentate may be treated so as to remove some of these substances such as cell membranes but retain other substances such as small molecules including, without limitation, organic acids and/or small peptides.

Accordingly, in some embodiments, the cellular mass component may include antimicrobial substances (i.e., antimicrobial peptides), antifungal substances (i.e. antifungal peptides) or any combinations thereof. Antifungal activity is likely linked to a protein produced by certain identified *Bacillus* strains. Treatment of *Bacillus* ferments with protease enzymes such as proteinase K, papain, pepsin and trypsin showed a dramatic reduction in inhibitory activity against indicator yeast species compared to untreated ferment. Treatment with enzymes that target starches (amylase) or fats (lipase) did not result in as substantial a reduction in activity.

Such antibacterial and antifungal substances are metabolites that have antibacterial and/or antifungal activity at a broad range of pH including above 5.5. The antibacterial substances may be at a concentration in the disclosed fermentates between about 0.01 mM and about 500 mM or any range therein. The antibacterial substances may be at a concentration in the disclosed fermentates between about 0.01 mM and about 500 mM or any range therein. As used herein, a "fermenting microorganism" refers to a microorganism that can ferment a carbohydrate source to antimicrobial substances. In the present disclosure, the fermenting microorganism may be a strain from the genus *Bacillus.* Suitable fermenting microorganisms may include, without limitation, strains from the species *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis, Bacillus toyonensis* or another GRAS species of *Bacillus.*

As used herein, a "substrate" is any substance providing a carbon source for the fermenting organism and is preferably selected from the group consisting of: sucrose, dextrose, lactose, starches, fruits, vegetables, whole grains, whey, milk or extracts thereof. The fruits may be selected from any of apples, watermelons, oranges, pears, strawberries, grapes, plum, mangos, blueberries, papaya, apricots, mandarins, bananas, grapefruit, lemons, limes, pineapples, jackfruits, melons, coconuts, avocados, peaches, kiwi, blackcurrants, blackberries, cherries, figs, nectarines, passionfruit, quince, raspberries, tangerines, pomegranates, mulberries, starfruit, guava, pomelos, cranberries, cantaloupe, tamarinds, persimmons, or a combination thereof. The vegetables may be selected from beets, cassavas, parsnips, beans, legumes, lentils, peas, potatoes, sweet potatoes, corn, plantains, taro, pumpkins, squash, turnips, or a combination thereof. The whole grains may be selected from any of wheat, barley, rice, rye, or a combination thereof.

In some embodiments, the disclosed fermentates have the ability to inhibit the growth of a contaminating microorganism by 100%, 90%, 80%, 70%, 60%, or 50% when diluted to less than 10%, 8%, 5%, 4%, 3%, 2%, or 1% (weight/volume of final product formulation (w/v)). To determine the antimicrobial activity of the fermentate, the contaminating microorganism may be grown in appropriate growth media to a sufficient level and diluted to between 0.5-1.0 McFarland. 10% (1 gram) of fermentate material is added to 10 ml of appropriate growth media for the contaminating microorganism strain. The sample is mixed and 5 mls may be transferred to a new tube containing 5 mls of growth media. This dilution process continues until there are treated tubes with fermentate concentrations ranging from 10% to 0.039% at half-fold intervals (10%, 5%, 2.5%, 1.25%, 0.625%, 0.312%, 0.156%, 0.078%, 0.039%). Approximately 1% (v/v) from the diluted culture sample was added to all the fermentate tubes. It is also added to one 5 ml tube that contains growth media but no fermentate (positive control). After 24 hours the growth of the positive control tube is compared to the growth in the fermentate tubes by measuring their optical densities, recorded at 600 nm. A fermentate with sufficient antimicrobial activity will be able to inhibit the growth of the contaminating microorganism by 100%, 90%, 80%, 70%, 60%, or 50% when diluted to less than 10%, 8%, 5%, 4%, 3%, 2%, 1%, 0.5%, 0.1%, or 0.05% (w/v).

In some embodiments, the fermentates may be further processed to produce a concentrated liquid or a dry powder. Methods of concentrating fermentates to produce concentrated liquids and/or dry powders are generally known in the art. For example, the disclosed fermentates may be evaporated using a falling film or similar system or may be spray-dried on a Buchi B-290 spray dryer,

### Methods for Producing a Fermentate

In another aspect, the present invention relates to methods for producing a fermentate. The methods may include mixing substrates with a fermenting microorganism selected from one of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis, Bacillus toyonensis* or another GRAS species of *Bacillus,* optionally water, and optionally a growth media capable of supporting the growth of the fermenting microorganism to produce a liquid composition, and incubating the liquid composition at a controlled temperature in a high oxygen environment to produce a fermentate.

Optionally, in certain embodiments, the fermenting microorganism is selected from one of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis,* and *Bacillus toyonensis.* Optionally, in some embodiments, the strain of *Bacillus* does not produce organic acids at a level effective to inhibit the growth of microorganisms. In other optional embodiments, the fermentate exhibits antibacterial and antifungal activity. In yet another optional embodiment, the fermentate exhibits antibacterial and antifungal activity at a pH above 5.5. Optionally, in certain embodiments, the fermenting microorganism is a *Bacillus amyloliquefaciens* strain. In certain embodiments, the strain of *Bacillus amyloliquefaciens* may be strain 723.

In some embodiments, the methods for producing a fermentate may further include evaporating the fermentate to produce a second fermentate. Methods of evaporating fermentates to produce concentrated liquids are generally known in the art. For example, evaporation step may be performed using a falling film or similar system.

In some embodiments, the methods for producing a fermentate may further include spray-drying the second fermentate to produce a powdered fermentate. Methods of spray-drying fermentates are generally known in the art. For example, the present inventors disclose in the non-limiting Examples that the disclosed fermentates may be spray-dried on a Buchi B-290 spray dryer.

In the present application, fermentation is aerobic. Fermentation may be achieved starting with dissolved oxygen concentration in liquid media between 40% and 100%, aerated consistently by filtered atmospheric air and mechanical agitation between 50-500 RPM during fermentation.

The "controlled temperature" of the present methods may be between about 10°C and about 60°C or any range therein. Suitably, the controlled temperature may is between about 10°C and about 50°C.

The "high oxygen environment" of the present methods may be a liquid environment where the dissolved oxygen concentration in liquid is between 40% and 100%. In other embodiments, the "high oxygen environment" contemplates a dissolved oxygen concentration in liquid between 50% and 99%, and the liquid is aerated consistently by filtered atmospheric air and mechanical agitation between 50-500 RPM during incubation. In still other embodiments, the "high oxygen environment" contemplates a dissolved oxygen concentration in liquid between 60% and 90%, or above 65%.

The present application contemplates a method for producing a fermentate with a fermenting organism selected from one of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis, Bacillus toyonensis* or another GRAS species of *Bacillus,* a substrate, optionally water, and a optionally a growth media capable of supporting the growth of the with the strain of *Bacillus* to produce a liquid composition; and incubating the liquid composition at a controlled temperature in a high oxygen environment to produce a fermentate. Optionally, in certain embodiments, the fermenting microorganism is selected from one of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis,* and *Bacillus toyonensis.* In certain embodiments, the fermenting microorganism is selected from a strain of *Bacillus amyloliquefaciens.* The strain of *Bacillus amyloliquefaciens* may be strain 723. The fermentate may include a cellular mass component with at least one metabolite that is an antimicrobial substance (i.e., antimicrobial peptides), antifungal substance (i.e. antifungal peptides) or any combinations thereof. The controlled temperature may be between about 10°C and about 50°C, and the high oxygen environment may be a liquid environment where the dissolved oxygen concentration in liquid is between 40% and 100%; however, such ranges may be further refined as described herein. The fermentate has the ability to inhibit the growth of a contaminating microorganism up to 100% when diluted to less than 5% (w/v).

The method for producing a fermentate may further include a step of evaporating the fermentate to produce a second fermentate. The method may also include a step of spray-drying the second fermentate to produce a powdered fermentate.

Optionally, in some embodiments, the strain of *Bacillus* does not produce organic acids at a level effective to inhibit the growth of microorganisms. In other optional embodiments, the fermentate exhibits antibacterial and antifungal activity. In yet another optional embodiment, the fermentate exhibits antibacterial and antifungal activity at a pH above5.5.

### Food Products

In a further aspect of the present invention, food products are provided. The food products may include any one of the fermentates disclosed herein or any one of the fermentates made by the methods disclosed herein. In one embodiment, the food product comprises a fermentate having a cellular mass component from a fermenting organism selected from one of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis, Bacillus toyonensis* or another GRAS species of *Bacillus,* and a substrate. Surprisingly, as reported in the non-limiting Examples, the present inventors demonstrate that fermentates produced using a fermenting organism selected from one of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis, Bacillus toyonensisor* another GRAS species of *Bacillus* displayed significant antimicrobial and antifungal activity against various microorganisms at a pH greater than 5.5.

As used herein, a "food product" may include any food product susceptible to microbial or fungal contamination or degradation. In some embodiments, the food product may be any food product that has a water activity greater than 0.2, 0.3, 0.35, 0.4, 0.45, 0.5, 0.55, 0.6, 0.65, 0.7, 0.75, 0.8, 0.85, or 0.9. Suitable food products may include, without limitation, culinary items, bakery items, cereals, pasta, meats, dairy items, rice, fish, nuts, beverages, confections, pet food, fruits, and vegetables.

Bakery items may include, without limitation, Breads, buns, rolls, Quick breads (biscuits, muffins, tortillas, cornbread, etc.), Sweet goods (cakes, brownies, cookies, pies, etc.), or Bakery Fillings (dairy-based, fruit-based, etc.).

Meats may include, without limitation, Cured Meats, Raw Beef / Pork (ground meat, whole muscle, etc.), Raw Poultry (ground poultry, whole muscle, etc.), Fermented meats, Emulsified meats (hot dogs, etc.), or Dried Meats.

Culinary items may include, without limitation, Dressings, Condiments, Mayonnaise, Sauces and gravies, Soups, Ready to eat dips, salsa, spreads, Ready to eat side items (coleslaw, potato salad, chicken salad, etc.), Ready to eat meals (lasagna, casserole, pasta dishes, etc.), jams, jellies, marmalades, fruit fillings, Desserts and puddings, or Syrups.

Beverages may include, without limitation, Teas, Coffee and coffee-based drinks, Fruit and vegetable juices, Fermented beverages, Beverage concentrates, Soft drinks, Acidified milk drinks and milk-based beverages, Carbonated soft drinks, Drink mixers (base used for bloody Mary's, margaritas, cocktails, etc.), Beer, or Wine.

Confections may include, without limitation, Chocolate and chocolate-based confections, Cakes, cookies, and other sweet treats.

Dairy items may include, without limitation, Fresh fermented dairy (cottage cheese, cream cheese, etc.), Dairy-based drinks (yogurt drinks, high-protein dairy drinks, etc.), Flavored milks, Cheese (shredded cheese, cheese blocks, etc.), Whipped toppings, Dairy-based desserts (flan, custard, pudding, etc.), Dairy-based dips (sour cream-based, Greek yogurt-based, etc.), Butter and spreads.

Pet food may include, without limitation, Kibble, Low- and high-moisture treats, Refrigerated rolls (meat rolls, veggie rolls, etc.), Palatants and flavor-enhancers, Broths, or Jerky.

In some embodiments, the food product may have a pH between about 1 and about 14, about 3 and about 14, about 4 and about 14, about 5 and about 15, or about 6 and about 14.

Additionally, the present application contemplates a food product including a fermentate having a cellular mass component from a fermenting organism selected from one of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis, Bacillus toyonensis* or another GRAS species *of Bacillus* and a substrate. The food product may have a pH between about 3 and about 14, preferably between 4 and 14, or at any sub-range between 3 and 14. The fermentate is particularly effective at a pH above 5.5. In certain embodiments, the food product has a water activity greater than 0.6. The food product may be culinary items, bakery items, cereals, pasta, meats, dairy items, rice, fish, nuts, beverages, confections, pet food, fruits, or vegetables. In certain embodiments, he food product has a volume and includes the fermentate in a concentration between about 0.1% and about 5% of the food product volume, or at any range therein as described elsewhere in this application. The fermentate of the food product may be a concentrated liquid or a dry powder, and preferably has the ability to inhibit the growth of a contaminating microorganism up to 100% when diluted to less than 5% (w/v).

### Methods for Killing or Inhibiting the Growth of a Microorganism on or within a Food Product

In a still further aspect, methods for killing or inhibiting the growth of a contaminating microorganism on or within a food product are provided. The food product has a volume, and the method contemplates the steps of making or obtaining a fermentate comprising a cellular mass component from a fermenting microorganism, and applying an effective amount of the fermentate to the food product so as to kill or inhibit the growth of the contaminating microorganism on or within the food product. The fermenting organism is selected from one of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis, Bacillus toyonensis* or another GRAS species of *Bacillus.* In certain embodiments, the fermenting organism selected from one of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis, and Bacillus toyonensis.* In other embodiments, the fermenting organism is a strain of *Bacillus amyloliquefaciens.* The fermentate is preferably applied in a concentration between about 0.08% and about 10% of the food product volume, but any value within that range may be selected, and any sub-range may be applicable as well. For example, the concentration may be between about 0.1 and about 5%, between about 0.2% and about 5%, between about 0.3% and about 5%, between about 0.6% and about 5%, between about 0.6% and about 2.5%, between about 1.25% and about 5%, and between about 1.25% and about 2.5%. The fermentate may be a concentrated liquid or a dry powder and has the ability to inhibit the growth of a contaminating microorganism by 100% when diluted to less than 5% (w/v).

The food product used in the method may be selected from the group consisting of culinary items, bakery items, cereals, pasta, meats, dairy items, rice, fish, nuts, beverages, confections, pet food, fruits, and vegetables. The contaminating microorganism may be a yeast species, a mold species, gram positive bacteria, or gram-negative bacteria. In certain embodiments, the strain of *Bacillus amyloliquefaciens* is strain 723. In certain embodiments, the strain of *Bacillus* does not produce organic acids at a level effective to inhibit the growth of microorganisms, and in other embodiments, the fermentate exhibits antibacterial and antifungal activity. In still other embodiments, the fermentate may exhibit antibacterial and antifungal activity at a pH above 5.5.

The cellular mass component may include at least one metabolite that is an antimicrobial substance (i.e., antimicrobial peptides), antifungal substance (i.e. antifungal peptides) or any combinations thereof.

"Effective amount" is intended to mean an amount of a fermentate described herein sufficient to inhibit the growth of a contaminating microorganism on a food product by, for example, 10%, 20%, 50%, 75%, 80%, 90%, 95%, or 1-fold, 3-fold, 5-fold, 10-fold, 20-fold, or more compared to a negative control. In some embodiments, the effective amount of a fermentate may be between about 0.1% and about 10% or any range therein. A "negative control" refers to a sample that serves as a reference for comparison to a test sample. For example, a test sample can be taken from a test condition including the presence of a fermentate and compared to negative control samples lacking the fermentate. One of skill in the art will recognize that controls can be designed for assessment of any number of parameters.

The antagonistic ability of known bioactive metabolites is dependent on the pH of the food matrix, especially in the case of organic acids such as propionic acid, produced during the fermentation of *Propionibacterium,* or sorbic acid derived from chemical synthesis or rowan berries. These acids force bacteria and fungi to devote energy to balancing free proton distribution across their membranes and the environment, leaving less for growth and proliferation, keeping their numbers low compared to untreated food systems. Because this antagonistic mechanism is based upon the acid's pKa, they are only effective in low-pH foods or systems that can be acidified to a low target pH. Therefore, these compounds are not effective methods of preservation for systems that cannot tolerate acidification, either because the food system is innately neutral or because the addition of acid would lead to an unacceptable change in flavor or another sensory quality. There are also spoilage microorganisms that are resistant to organic acids, even at low pHs. Acid preservative-tolerant yeasts, such as *Zygosaccharomyces spp.* are one such group of organisms.

Nisin is a highly effective antibacterial protein produced by *Lactococcus lactis* that can be used in relatively neutral pH food systems. There are, however, regulatory limits to the use of purified nisin preparations and nisin exhibits no antagonistic action against fungal spoilage organisms. Natamycin is another naturally derived peptide, produced though the fermentation of *Streptomyces natalensis,* that does exhibit antifungal properties. However, it has no antibacterial effects and does present some allergen and regulatory concerns as a food additive. Moreover, many existing industry solutions for control of fungal organisms in food systems, such as sorbates and benzoates, can impart a harsh, chemical flavor to the food. Other flavorless chemical preservatives like sulfites, nitrates and nitrites commonly used in many cured meat products have strict usage limits. Organic acid salts, including calcium propionate and sodium lactate, are also used in meat products in addition to baked goods, beverages, and dairy products but are not preferred as label ingredients. Customers have a negative view of these chemical preservatives, and they are not recognized for use in products with 'natural' branding.

Current natural fermentation relies on organic acid and/or antimicrobial peptides to hold back spoilage in finished foods. Organic acids produced in fermentates only work well in low pH foods (<5.5). The is because as soon as the pH reaches >5.0, the percent of undissociated acid is significantly reduced and is no longer effective. The *Bacillus* strains disclosed herein lower pH during fermentation and may produce a range of organic acids, but final fermentation product maintains a neutral pH and activity in neutral pH applications. Given the increasing customer and industry demand for natural preservative solutions, the limitations of the commonly used peptides and organic acids currently available provides an opportunity for a natural preservative that is effective against bacterial and fungal spoilage across a wide range of pHs, specifically neutral pH (>5.5).

The present disclosure ferments strains of *Bacillus* that do not produce organic acids at a level effective to inhibit the growth of microorganisms, but do contain metabolites that have antibacterial and antifungal activity at a broad range of pH including >5.5.

Overall, the present invention covers the following aspects:
Aspect 1: A method for producing a fermentate comprising:
   (a) obtaining a substrate;
   (b) mixing the substrate with a strain of *Bacillus* selected from a group consisting of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis, Bacillus toyonensis,* and a GRAS species of *Bacillus* to produce a composition; and
   (c) incubating the composition at a controlled temperature in a high oxygen environment to produce a fermentate.
Aspect 2: The method of aspect 1, wherein the strain of *Bacillus* does not produce organic acids at a level effective to inhibit the growth of microorganisms.
Aspect 3: The method of aspect 1, wherein the fermentate exhibits antibacterial and antifungal activity.
Aspect 4: The method of aspect 2, wherein the fermentate exhibits antibacterial and antifungal activity at a pH above 5.5.
Aspect 5: The method of aspect 1, wherein the strain of *Bacillus* is selected from a group consisting of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis,* and *Bacillus toyonensis.*
Aspect 6: The method of aspect 5, wherein the strain of *Bacillus* is a *Bacillus amyloliquefaciens* strain.
Aspect 7: The method of aspect 6, wherein the strain of *Bacillus amyloliquefaciens* is strain 723.
Aspect 8: The method of aspect 1, wherein the composition is a liquid composition and the method further comprises a step of evaporating the fermentate to produce a second fermentate.
Aspect 9: The method of aspect 8, further comprising a step of spray-drying the second fermentate to produce a powdered fermentate.
Aspect 10: The method of aspect 1, wherein the controlled temperature is between about 10°C and about 50°C.
Aspect 11: The method of aspect 1, wherein the high oxygen environment is a liquid environment where the dissolved oxygen concentration in liquid is between 40% and 100%.
Aspect 12: The method of aspect 11, wherein the dissolved oxygen concentration in liquid is between 50% and 99%, and the liquid is aerated consistently by filtered atmospheric air and mechanical agitation between 50-500 RPM during incubation.
Aspect 13: A method for killing or inhibiting the growth of a contaminating microorganism on or within a food product, the food product having a volume, and the method comprising:
   making or obtaining a fermentate comprising a cellular mass component from a fermenting microorganism, and a substrate; and
   applying an effective amount of the fermentate to the food product so as to kill or inhibit the growth of the contaminating microorganism on or within the food product;
   wherein the fermenting organism is a strain of *Bacillus* selected from a group consisting of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis, Bacillus toyonensis* and a GRAS species of *Bacillus.*
Aspect 14: The method of aspect 13, wherein the fermentate is applied in a concentration between about 0.1% and about 5% of the food product volume.
Aspect 15: The method of aspect 13, wherein the food product is selected from the group consisting of culinary items, bakery items, cereals, pasta, meats, dairy items, rice, fish, nuts, beverages, confections, animal feed, fruits, and vegetables.
Aspect 16: The method of aspect 13, wherein the contaminating microorganism is selected from the group consisting of a yeast species, a mold species, a gram-positive bacteria, and a gram-negative bacteria, or any combination thereof.
Aspect 17: The method of aspect 13, wherein the strain of *Bacillus* is selected from a group consisting of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis,* and *Bacillus toyonensis.*
Aspect 18: The method of aspect 17, wherein the strain of *Bacillus* is a strain of *Bacillus amyloliquefaciens.*
Aspect 19: The method of aspect 18, wherein the strain of *Bacillus* is *Bacillus amyloliquefaciens* strain 723.
Aspect 20: The method of aspect 13, wherein the strain of *Bacillus* does not produce organic acids at a level effective to inhibit the growth of microorganisms.
Aspect 21: The method of aspect 13, wherein the fermentate exhibits antibacterial and antifungal activity.
Aspect 22: The method of aspect 21, wherein the fermentate exhibits antibacterial and antifungal activity at a pH above 5.5.
Aspect 23: The method of aspect 13, wherein the substrate provides a carbon source for the fermenting organism.
Aspect 24: The method of aspect 23, wherein the substrate is selected from the group consisting of: sucrose, dextrose, lactose, starches, fruits, vegetables, whole grains, whey, milk or extracts thereof.
Aspect 25: The method of aspect 13, wherein the fermentate is a concentrated liquid.
Aspect 26: The method of aspect 13, wherein the fermentate is a dry powder.
Aspect 27: A fermentate produced by any one of the methods of aspects 1 to 26.
Aspect 28: A food product comprising a fermentate having a cellular mass component from a strain of *Bacillus* selected from a group consisting of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis, Bacillus toyonensis* and a GRAS species of *Bacillus,* and a substrate.
Aspect 29: The food product of aspect 28, wherein the strain of *Bacillus* is selected from a group consisting of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis,* and *Bacillus toyonensis.*
Aspect 30: The food product of aspect 29, wherein the strain of *Bacillus* is a strain of *Bacillus amyloliquefaciens.*
Aspect 31: The food products of aspect 30, wherein the strain of *Bacillus* is *Bacillus amyloliquefaciens* strain 723.
Aspect 32: The food product of aspect 28, wherein the food product has a pH above 5.5.
Aspect 33: The food product of aspect 28, wherein the food product is selected from the group consisting of culinary items, bakery items, cereals, pasta, meats, dairy items, rice, fish, nuts, beverages, confections, pet food, fruits, and vegetables.
Aspect 34: The food product of aspect 28, wherein the food product has a volume and includes the fermentate in a concentration between about 0.1% and about 5% of the food product volume.
Aspect 35: The method of aspect 28, wherein the strain of *Bacillus* does not produce organic acids at a level effective to inhibit the growth of microorganisms.
Aspect 36: The method of aspect 28, wherein the fermentate exhibits antibacterial and antifungal activity.
Aspect 37: The food product of aspect 28, wherein the the substrate is selected from the group consisting of: sucrose, dextrose, lactose, starches, fruits, vegetables, whole grains, whey, milk or extracts thereof.
Aspect 38: The food product of aspect 28, wherein the fermentate is a concentrated liquid.
Aspect 39: The food product of aspect 28, wherein the fermentate is a dry powder.
Aspect 40: The food product of aspect 28, wherein the fermentate has the ability to inhibit the growth of a contaminating microorganism up to 100% when diluted to less than 5% (w/v).

### Illustrative Embodiments

Embodiment 1: a fermentate comprising a cellular mass component selected from one of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis, Bacillus toyonensis* or another GRAS species of *Bacillus* and a substrate.

Embodiment 2: the fermentate of embodiment 1, wherein the fermenting organism is selected from one of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis,* and *Bacillus toyonensis.*

Embodiment 3: the fermentate of embodiment 1, wherein the fermenting organism is a *Bacillus amyloliquefaciens* strain.

Embodiment 4: the fermentate of embodiment 3, wherein the strain of *Bacillus amyloliquefaciens* is strain 723.

Embodiment 5: the fermentate of any one of the preceding embodiments, wherein the cellular mass component comprises at least one metabolite selected from the group consisting of an antimicrobial substance (i.e., antimicrobial peptides), and antifungal substance (i.e. antifungal peptides) and combinations thereof.

Embodiment 6: the fermentate of any one of the preceding embodiments, wherein the substrate provides a carbon source for the fermenting organism.

Embodiment 7: the fermentate of any one of the preceding embodiments, wherein the strain of *Bacillus* does not produce organic acids at a level effective to inhibit the growth of microorganisms.

Embodiment 8: the fermentate of any one of the preceding embodiments, wherein the fermentate exhibits antibacterial or antifungal activity.

Embodiment 9: the fermentate of any one of the preceding embodiments, wherein the fermentate exhibits antibacterial and antifungal activity.

Embodiment 11: the fermentate of any one of the preceding embodiments, wherein the fermentate exhibits antibacterial or antifungal activity at a pH above 5.5.

Embodiment 11: the fermentate of any one of the preceding embodiments, wherein the fermentate exhibits antibacterial and antifungal activity at a pH above 5.5.

Embodiment 12: the fermentate of any one of the preceding embodiments, wherein the fermentate has the ability to inhibit the growth of a contaminating microorganism up to 100% when diluted to less than 5% (w/v).

Embodiment 13: the fermentate of any one of the preceding embodiments, wherein the fermentate is a concentrated liquid.

Embodiment 14: the fermentate of any one of the preceding embodiments, wherein the fermentate is a dry powder.

Embodiment 15: a method for producing a fermentate comprising: (a) obtaining a fermenting organism selected from one of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis, Bacillus toyonensis* or another GRAS species of *Bacillus* and a substrate and media substrate capable of supporting the growth of the fermenting microorganism; (b) combining the fermenting organism with the substrate to create a fermenting mixture; and (c) incubating the fermenting mixture at a at a controlled temperature in a high oxygen environment to produce a fermentate.

Embodiment 16: the method of embodiment 15, wherein the fermenting mixture is a liquid composition and the method further comprises evaporating the fermentate to produce a second fermentate.

Embodiment 17: the method of embodiment 13, further comprising spray-drying the second fermentate to produce a powdered fermentate.

Embodiment 18: the method of any one of embodiments 15-17, wherein the fermenting organism selected from one of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis,* and *Bacillus toyonensis.*

Embodiment 19: the method of any one of embodiments 15- 18, wherein the fermenting organism is a strain of *Bacillus amyloliquefaciens.*

Embodiment 20: the method of any one of embodiments 15-19, wherein the strain of *Bacillus amyloliquefaciens* is strain 723.

Embodiment 21: the method of embodiment 15-20, wherein the strain of *Bacillus* does not produce organic acids at a level effective to inhibit the growth of microorganisms.

Embodiment 22: the method of embodiment 15-21, wherein the fermentate exhibits antibacterial or antifungal activity.

Embodiment 23: the method of embodiment 15-22, wherein the fermentate exhibits antibacterial and antifungal activity.

Embodiment 24: the method of embodiment 15-23, wherein the fermentate exhibits antibacterial or antifungal activity at a pH above 5.5.

Embodiment 25: the method of embodiment 15-25, wherein the fermentate exhibits antibacterial and antifungal activity at a pH above 5.5.

Embodiment 26: the method of any one of embodiments 15-25, wherein the controlled temperature is between about 10°C and about 50°C.

Embodiment 27: the method of any one of embodiments 15-26, wherein the high oxygen environment is a liquid environment where the dissolved oxygen concentration in liquid is between 40% and 100%.

Embodiment 28: a food product comprising any one of the fermentates of embodiments 1-14 or any one of the fermentates made by the methods of embodiments 15-27.

Embodiment 29: the food product of embodiment 28, wherein the food product has a pH between about 3 and about 14.

Embodiment 30: the food product of any one of embodiments 28-29, wherein the food product has a water activity greater than 0.6.

Embodiment 31: the food product of any one of embodiments 28-30, wherein the food product is selected from the group consisting of culinary items, bakery items, cereals, pasta, meats, dairy items, rice, fish, nuts, beverages, confections, pet food, fruits, and vegetables.

Embodiment 32: a method for killing or inhibiting the growth of a contaminating microorganism on or within a food product comprising: making or obtaining any one of the fermentates of embodiments 1-14 or performing any one of the methods of embodiments 15-26; and applying an effective amount of the fermentate to the food product so as to kill or inhibit the growth of the contaminating microorganism on or within the food product.

Embodiment 33: the method of embodiment 32, wherein the fermentate is applied in an amount between about 0.1% and about 5%.

Embodiment 34: the method of any one of embodiments 32-33, wherein the contaminating microorganism is selected from the group consisting of a yeast species, a mold species, gram-positive bacteria, and a gram-negative bacteria.

Embodiment 35: the method of any one of embodiments 32-34, wherein the food product is selected from the group consisting of culinary items, bakery items, cereals, pasta, meats, dairy items, rice, fish, nuts, beverages, confections, pet food, fruits, and vegetables.

Embodiment 36: a fermentate produced by any one of the methods of embodiments 15-27.

The present disclosure is not limited to the specific details of construction, arrangement of components, or method steps set forth herein. The compositions and methods disclosed herein are capable of being made, practiced, used, carried out and/or formed in various ways that will be apparent to one of skill in the art in light of the disclosure that follows. The phraseology and terminology used herein is for the purpose of description only and should not be regarded as limiting to the scope of the claims. Ordinal indicators, such as first, second, and third, as used in the description and the claims to refer to various structures or method steps, are not meant to be construed to indicate any specific structures or steps, or any particular order or configuration to such structures or steps. All methods described herein can be performed in any suitable order unless otherwise indicated herein or otherwise clearly contradicted by context. The use of any and all examples, or exemplary language (e.g., "such as") provided herein, is intended merely to facilitate the disclosure and does not imply any limitation on the scope of the disclosure unless otherwise claimed. No language in the specification, and no structures shown in the drawings, should be construed as indicating that any non-claimed element is essential to the practice of the disclosed subject matter. The use herein of the terms "including," "comprising," or "having," and variations thereof, is meant to encompass the elements listed thereafter and equivalents thereof, as well as additional elements. Embodiments recited as "including," "comprising," or "having" certain elements are also contemplated as "consisting essentially of" and "consisting of" those certain elements.

Recitation of ranges of values herein are merely intended to serve as a shorthand method of referring individually to each separate value falling within the range, unless otherwise indicated herein, and each separate value is incorporated into the specification as if it were individually recited herein. For example, if a concentration range is stated as 1 mM to 50 mM, it is intended that values such as 2 mM to 40 mM, 10 mM to 30 mM, or 1 mM to 3 mM, etc., are expressly enumerated in this specification. These are only examples of what is specifically intended, and all possible combinations of numerical values between and including the lowest value and the highest value enumerated are to be considered to be expressly stated in this disclosure. Use of the word "about" to describe a particular recited amount or range of amounts is meant to indicate that values very near to the recited amount are included in that amount, such as values that could or naturally would be accounted for due to manufacturing tolerances, instrument and human error in forming measurements, and the like. All percentages referring to amounts are by weight unless indicated otherwise.

No admission is made that any reference, including any non-patent or patent document cited in this specification, constitutes prior art. In particular, it will be understood that, unless otherwise stated, reference to any document herein does not constitute an admission that any of these documents forms part of the common general knowledge in the art in the United States or in any other country. Any discussion of the references states what their authors assert, and the applicant reserves the right to challenge the accuracy and pertinence of any of the documents cited herein. All references cited herein are fully incorporated by reference in their entirety, unless explicitly indicated otherwise. The present disclosure shall control in the event there are any disparities between any definitions and/or description found in the cited references.

Unless otherwise specified or indicated by context, the terms "a", "an", and "the" mean "one or more." For example, "a protein" or "an RNA" should be interpreted to mean "one or more proteins" or "one or more RNAs," respectively.

The following examples are meant only to be illustrative and are not meant as limitations on the scope of the invention or of the appended claims.

### EXAMPLES

### Example 1: General Methods

### Standard Enriched Media Method:

Target bacteria strains were grown from frozen colony stocks (tryptic soy broth + 10% glycerol, stored at -80C) picked into 10 milliliters (ml) of appropriate growth media (tryptic soy broth) and grown for ~24 hours (hrs) in a shaking incubator (150 rpm) at 32°C. An aseptic 1% volumetric transfer was made from the overnight culture into a fresh 10 milliliters (ml) of tryptic soy broth and grown for ~24 hrs at 32°C, shaking. Following fermentation, the cultures were enumerated to determine CFU/ml (colony forming units/ml) using standard media and methods. A portion of the fermentation growth was filter-sterilized and tested for activity using various methods described in the following examples.

### Standard Shake Flask (Minimal Media) Method:

Target bacteria strains were grown from frozen colony stocks (tryptic soy broth + 10% glycerol, stored at -80C) picked into 10 milliliters (ml) of appropriate growth media (tryptic soy broth) for ~24 hours (hrs) in a shaking incubator (150 rpm) at 32°C. An aseptic 1% volumetric transfer was made from the overnight culture into individual 125ml Erlenmeyer flask sterilized with 75ml of the appropriate minimal media components (yeast protein/s, salt/s, trace element/s and carbohydrate source/s, in sterile deionized water). The inoculated flasks were incubated in a 32°C shaking (150 rpm) incubator and grown for 12-24 hours. Following fermentation, the cultures were enumerated to determine CFU/ml (colony forming units/ml) using standard media and methods. A portion of the fermentation growth was filter-sterilized and tested for activity using various methods described in the following examples.

### Laboratory Pilot-Scale Fermenter Method:

Target bacteria strains were grown from frozen colony stocks (tryptic soy broth + 10% glycerol, stored at -80C) picked into 10 milliliters (ml) of appropriate growth media (tryptic soy broth) and grown for 18-24 hours (hrs) in a shaking incubator (150 rpm) at 32°C. An aseptic 1% volumetric transfer was made from the overnight culture into a fresh 10 milliliters (ml) of appropriate growth media (tryptic soy broth) and grown for between 6-12 hours. An aseptic 2% volumetric transfer was made from 6-12 hour culture into fermentation vessel containing appropriate minimal media components and grown for 8-24 hours.

### Pilot Scale Spray Dry Ferment Method:

1-5% w/v maltodextrin was dissolved into liquid fermentate from pilot-scale fermenter and dried using a Buchi B-290 pilot-scale spray drier at temperatures between 140 and 200°C.

### DNA Sequencing:

Genus and species of bacteria were determined by 16s rDNA sequencing. Bacterial genomic DNA was extracted from pure culture using a commercial kit. The 16S gene target was amplified with an Applied Biosystems SimpliAmp Thermal Cycler using bacterial PCR primers 27F (5'-AGAGTTTGATYMTGGCTCAG-3') and 1492R (5'-TACCTTGTTAYGACTT-3'). PCR product quality was confirmed by agarose gel electrophoresis and sequenced via Sanger sequencing. The sequences were identified with the NCBI Blastn database with a percent identity greater than 97%.

### Example 2

*Bacillus* strain 723 was identified using 16s rRNA sequencing as *Bacillus amyloliquifaciens.* Antibacterial and antifungal activity of this this strain was compared to the activity of two similar strains, *Bacillus amyloliquifaciens* 12071 and *Bacillus valezensis* 2081113, a species closely related to *amyloliquifaciens.* The three strains were grown in enriched bacterial growth media for 24 hours. The resulting growth was centrifuged and filtered to obtain a cell free supernatant (CFS) containing all bioactive metabolites. The CFS of each strain was serially diluted from 50% to 0.78% and tested against gram positive *bacteria Micrococcus luteus,* strain B-287 for presence of antibacterial metabolites and yeast *Candida parapsilosis,* strain Y-619 for presence of antifungal metabolites.

The cell free supernatant of *B. amyloliquifaciens* strain 723 demonstrated unique antibacterial and antifungal effects even when compared to very closely related strains.

**Table 1. Percent inhibition of Micrococcus luteus (B-287), in tryptic soy broth (pH 7.2) in the presence Bacillus CFS.**

| B-287 | 2081113 | 12071 | 723 |
|---|---|---|---|
| 50.00% | 100.0% | 100.0% | 100.0% |
| 25.00% | 24.6% | 55.6% | 99.3% |
| 12.50% | 0.0% | 0.0% | 97.9% |
| 6.25% | 0.0% | 0.0% | 100.0% |
| 3.13% | 0.0% | 0.0% | 100.0% |
| 1.56% | 0.0% | 0.0% | 98.1% |
| 0.78% | 0.0% | 0.0% | 41.8% |

**Table 2. Percent inhibition of Candidaparapsilosis (Y-619), in potato dextrose broth (pH 5.2) in the presence of Bacillus CFS.**

| Y-619 | 2081113 | 12071 | 723 |
|---|---|---|---|
| 50.00% | 0.0% | 0.0% | 83.8% |
| 25.00% | 0.0% | 0.0% | 30.7% |
| 12.50% | 0.0% | 0.0% | 11.8% |
| 6.25% | 0.0% | 0.0% | 2.3% |
| 3.13% | 0.0% | 0.0% | 0.0% |
| 1.56% | 0.0% | 0.0% | 0.0% |
| 0.78% | 0.0% | 0.0% | 0.0% |

### Example 3

Multiple *Bacillus* species were grown in minimal growth media for 18-24 hours. Resulting growth was centrifuged and filtered to obtain a cell-free supernatant (CFS) containing all bioactive metabolites. The CFS of each strain was serially diluted from 50% to 0.78% and tested against yeast *Candida parapsilosis,* strain Y-619 for presence of antifungal metabolites and bacteria *Micrococcus luteus,* strain B-287 for presence of antibacterial metabolites.

The cell-free supernatant produced by *Bacillus* species exhibiting an activity greater than 50% when diluted to 25% or more are listed in table 3 for antifungal activity and table 4 for antibacterial activity. The *Bacillus* strains highlighted in yellow had activity against both yeast (Y-619) and bacteria (B-287).

**Table 3. Percent inhibition of Candida parapsilosis (Y-619), in potato dextrose broth (pH 5.2-5.4) in the presence of Bacillus CFS.**

| Y-619 | 16S Identification | 50.0% | 25.0% | 12.5% | 6.25% | 3.13% | 1.56% | 0.78% |
|---|---|---|---|---|---|---|---|---|
| 723 | Bacillus amyloliquefaciens | 97.0% | 96.5% | 94.0% | 88.1% | 72.2% | 54.3% | 17.6% |
| 12071 | Bacillus amyloliquefaciens | 98.4% | 95.8% | 95.3% | 87.5% | 74.4% | 47.9% | 5.2% |
| 283111 | Bacillus licheniformis | 89.3% | 79.4% | 71.1% | 57.0% | 25.1% | 3.1% | 0.0% |
| 17419 | Bacillus pumilus | 76.2% | 71.3% | 13.2% | 20.7% | 17.4% | 3.3% | 0.0% |
| 109140 | Bacillus siamensis | 91.8% | 96.0% | 89.8% | 81.2% | 63.7% | 32.0% | 0.0% |
| 12025 | Bacillus subtilis | 91.6% | 90.4% | 78.5% | 66.9% | 49.8% | 23.3% | 14.4% |
| 12026 | Bacillus subtilis | 65.1% | 81.3% | 84.1% | 76.8% | 61.1% | 37.0% | 0.0% |
| 12027 | Bacillus subtilis | 91.6% | 73.8% | 64.6% | 47.7% | 30.7% | 22.1% | 21.8% |
| 283114 | Bacillus subtilis | 97.5% | 97.6% | 91.4% | 79.1% | 64.8% | 42.5% | 0.0% |
| 18547 | Bacillus tequilensis | 93.2% | 77.2% | 70.8% | 54.5% | 17.4% | 0.0% | 0.0% |

**Table 4. Percent inhibition of Micrococcus luteus (B-287), in tryptic soy broth (pH 7.2) in the presence of Bacillus CFS.**

| B-287 | 16S Identification | 50.0% | 25.0% | 12.5% | 6.25% | 3.13% | 1.56% | 0.78% |
|---|---|---|---|---|---|---|---|---|
| 723 | Bacillus amyloliquefaciens | 74.3% | 66.5% | 58.6% | 23.2% | 0.0% | 0.0% | 0.0% |
| 12071 | Bacillus amyloliquefaciens | 80.0% | 85.3% | 92.4% | 79.7% | 16.7% | 0.0% | 0.0% |
| 17544 | Bacillus amyloliquefaciens | 76.7% | 92.2% | 96.0% | 97.2% | 78.1% | 55.9% | 27.5% |
| 17364 | Bacillus marisflavi | 55.8% | 81.8% | 0.0% | 2.4% | 0.0% | 0.0% | 0.0% |
| 17946 | Bacillus megaterium | 84.6% | 91.6% | 95.8% | 86.7% | 37.1% | 0.0% | 0.0% |
| 20027 | Bacillus paralicheniformis | 76.7% | 83.8% | 0.0% | 0.0% | 0.0% | 0.0% | 0.0% |
| 20029 | Bacillus paralicheniformis | 76.7% | 87.4% | 7.4% | 0.0% | 0.0% | 9.7% | 22.1% |
| 200121 | Bacillus paralicheniformis | 74.8% | 82.3% | 87.2% | 93.3% | 81.4% | 67.1% | 45.3% |
| 200221 | Bacillus paralicheniformis | 82.6% | 87.8% | 0.0% | 0.0% | 0.0% | 0.0% | 0.0% |
| 170411 | Bacillus safensis | 76.7% | 92.2% | 96.0% | 97.2% | 78.1% | 55.9% | 27.5% |
| 12025 | Bacillus subtilis | 84.1% | 61.3% | 0.0% | 0.0% | 0.0% | 0.0% | 8.1% |
| 12026 | Bacillus subtilis | 84.6% | 91.6% | 95.8% | 86.7% | 37.1% | 0.0% | 0.0% |
| 20017 | Bacillus toyonensis | 85.1% | 92.8% | 96.1% | 84.5% | 25.2% | 20.0% | 0.0% |
| 200110 | Bacillus toyonensis | 83.0% | 96.4% | 98.7% | 98.7% | 57.7% | 22.8% | 6.8% |

### Example 4

The *B. amyloliquifaciens* strain 723 liquid fermentate was spray-dried and a 2% aqueous solution was made in sterile, deionized water, filtered through 0.2um filter to remove any particulate matter, and tested *in vitro* for anti-bacterial activity against gram negative bacteria *Escherichia coli,* strain EC-112 and gram positive bacteria *Listeria innocua,* strain LI-3314 and *Micrococcus luteus,* strain B-287.

**Table 5. Percent inhibition of Escherichia coli (EC-112), Listeria innocua (LI-3314) and Micrococcus luteus (B-287), in tryptic soy broth (pH 7.2) in the presence of spray dried Bacillus fermentate product (BA).**

| BA @2% | EC-112 | LI-3314 | B-287 |
|---|---|---|---|
| 1.0% | 86.9% | 95.5% | 91.3% |
| 0.50% | 94.7% | 97.6% | 94.7% |
| 0.25% | 97.9% | 96.9% | 97.9% |
| 0.13% | 63.0% | 65.0% | 63.0% |
| 0.06% | 41.0% | 0.0% | 41.0% |
| 0.03% | 17.0% | 0.0% | 17.0% |
| 0.02% | 9.4% | 0.0% | 9.4% |

### Example 5

A 1% solution of *B. amyloliquifaciens* strain 723 spray dried product (BA) was prepared in sterile water. In addition, a 0.1% Potassium sorbate (Ps) and 1% organic acid-based fermentate (OA) was prepared in the same manner. All three solutions were tested against yeasts *Candida parapsilosis,* strain Y-619 and *Saccharomyces cerevisiae,* strain Y-1545, in growth media set to pH 4, 5, 6, and 7. The Bacillus (BA) product provided antifungal activity against both yeast indicators across a greater pH range than either the 1% organic acid-based preservative, (OA), or 0.1% potassium sorbate (Ps).

**Table 6. Percent inhibition of Candida parapsilosis, Y-619 grown at pH 4, 5, 6, and 7 in the presence of 0.1% potassium sorbate (Ps), 1% organic acid-based preservative (OA), and 1% B. amyloliquifaciens fermentate (BA).**

| y619 | Ps 0.1% | OA 1% | BA 1% |
|---|---|---|---|
| pH 4 | 100.0% | 97.4% | 85.9% |
| pH 5 | 100.0% | 98.1% | 87.4% |
| pH 6 | 100.0% | 40.4% | 89.8% |
| pH 7 | 55.6% | 21.6% | 79.4% |

**Table 7. Percent inhibition of Saccharomyces cerevisiae, Y-1545 grown at pH 4, 5, 6, and 7 in the presence of 0.1% Potassium sorbate (Ps), 1% organic acid-based preservative (OA), and 1% B. amyloliquifaciens fermentate (BA).**

| y1545 | Ps 0.1% | OA 1% | BA 1% |
|---|---|---|---|
| pH 4 | 100.0% | 99.2% | 91.8% |
| pH 5 | 100.0% | 25.2% | 96.4% |
| pH 6 | 99.3% | 0.0% | 95.4% |
| pH 7 | 0.0% | 0.0% | 57.0% |

### Example 6

The *B. amyloliquifaciens* strain 723 fermentate spray dried product was tested at 1% in a 10% rehydrated potato flake slurry with a pH of 6.0. The potato flake slurry was challenged with a cocktail of three yeasts, *Saccharomyces cerevisiae,* strain Y-1545, *Zygosaccharomyces rouxii,* strain Y-229, and *Zygosaccharomyces bailii,* strain Y-7239, at a combined dose of approximately 350 CFU/ml. These yeast species were chosen as indicators due to their documented resistance to weak acids and organic acid-based preservatives. The potato slurry was held at room temperature and analyzed over time for enumeration of yeast. Usage rates were in the range of typical inclusion in finished foods.

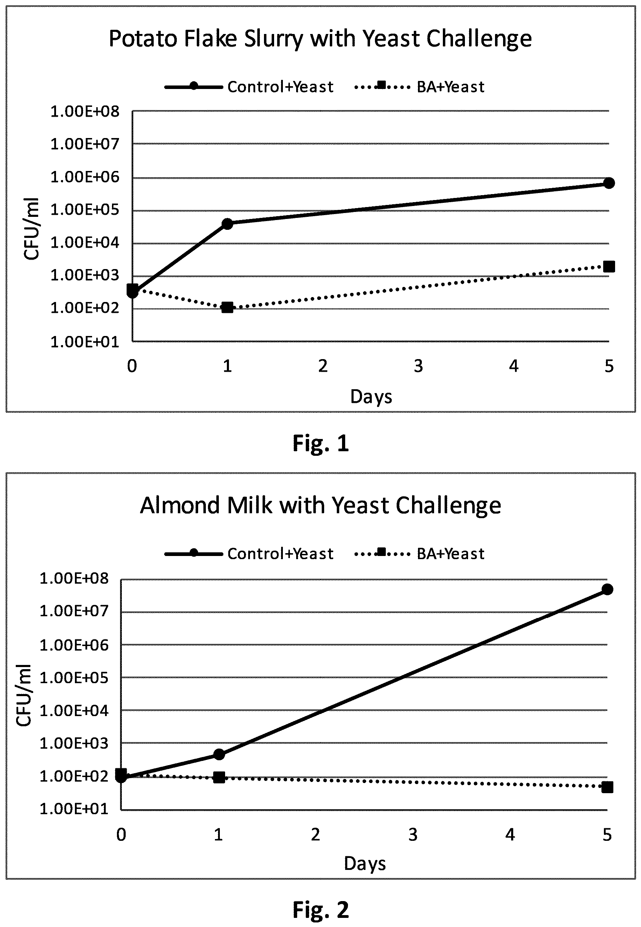

Referring now to Figure 1, therein growth of yeast in a 10% potato flake slurry treated with *B. amyloliquifaciens* strain 723 fermentate (1%) compared to an untreated control held at room temperature is shown. Growth of yeasts was both retarded and inhibited in samples of the potato slurry treated with 1% *B. amyloliquifaciens* strain 723 fermentate, resulting in a total growth reduction of more than two logs or 99% (2.0E+03 CFU/g) compared to untreated control samples (6.5E+05 CFU/g) at Day 5.

### Example 7

The *B. amyloliquifaciens* strain 723 fermentate spray dried product was tested at 1% in almond milk with a pH of 7.8. The almond milk was challenged with a cocktail of three yeasts, *Saccharomyces cerevisiae,* Y-1545, *Zygosaccharomyces rouxii,* Y-229, and *Zygosaccharomyces bailii,* Y-7239, at a combined dose of approximately 100 CFU/ml. The almond milk was held at refrigerated temperature and analyzed over time for enumeration of yeast. Usage rates were in range of typical inclusion in finished foods.

Referring now to Figure 2, therein the growth of yeast in almond milk treated with B. *amyloliquifaciens* strain 723 fermentate (1%) compared to an untreated control held at refrigerated temperature is demonstrated. Yeast growth was entirely inhibited in samples of almond milk treated with 1% *B. amyloliquifaciens* strain 723 fermentate. Yeast counts fell in treated samples over the course of 5 days (5.0E+01 CFU/ml) compared to untreated control samples, where yeast counts grew to 5.0E+07 CFU/ml by the conclusion of the study.

### Example 8

The *B. amyloliquifaciens* strain 723 fermentate spray dried product was tested in chicken salad which had a starting pH of 5.8. The chicken salad was freshly made and allowed to spoil naturally over time. To prevent the product from being spoiled by Lactic Acid Bacteria (LAB), which can cause an accumulation of lactic acid reducing the pH over time, Nisin preparation (500ppm) was added. Treatments included a no preservative Control, 500ppm Nisin, 0.1% Potassium sorbate + 500ppm Nisin (Ps+N), and 1% Bacillus fermentate + 500ppm Nisin (BA+N). Usage rates were in the range of typical inclusion in finished foods.

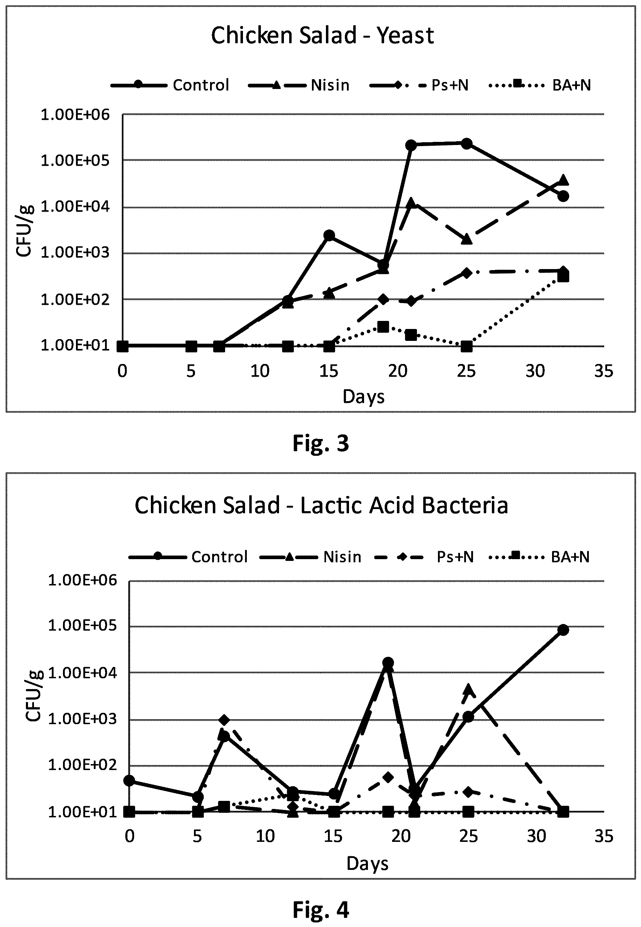

Referring now to Figures 3 and 4, therein the growth of yeast and Lactic Acid Bacteria in chicken salad treated with 1% BA + Nisin compared to Control, 500ppm Nisin, and 0.1% Potassium sorbate + Nisin is shown. Substantial yeast growth was not observed in chicken salad samples treated with 1% *Bacillus* fermentate until after Day 25, more than 10 days later than yeast growth was observed in other samples. Yeast counts (CFU/g) also rose much higher in untreated control and nisin control samples compared to *Bacillus*-treated samples. The study was ended after 32 days when lactic acid counts rose sharply in untreated control samples (contributing to the drop in pH and resulting yeast counts seen at the same time point)

### Example 9

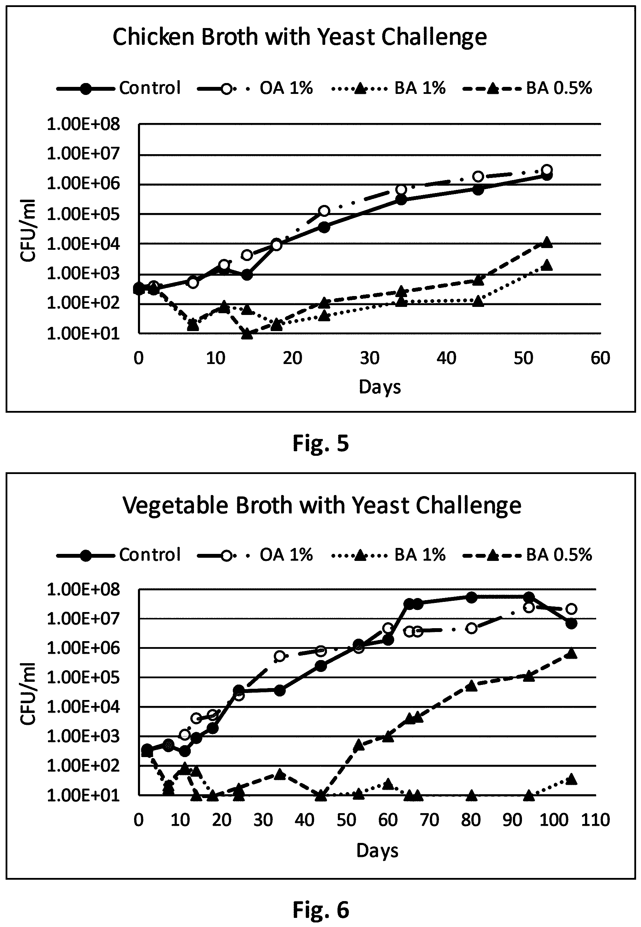

The *B. amyloliquifaciens* strain 723 fermentate spray dried product was tested at 1% and 0.5% in preservative-free chicken broth with a pH of 6.4 and compared to a Control and 1% organic acid-based preservative, (OA). The chicken broth was challenged with a cocktail of two yeasts, *Saccharomyces cerevisiae,* Y-1545, and *Torulaspora delbrueckii,* Y-866, at a combined dose of approximately 300 CFU/ml. The chicken broth was held at refrigerated temperature and analyzed over time for enumeration of yeast. Usage rates were in the range of typical inclusion in finished foods. Referring to Figure 5, yeast counts remained at or below inoculation levels through day 40 in samples treated with 1% or 0.5% BA. Comparatively yeast counts rose steadily in both control samples and samples treated with 1% organic acid-based preservative by day 40 (1.7E+05 CFU/ml and 1.8E+06 CFU/ml, respectively). Beginning at day 18, yeast counts were consistently higher in 0.5% BA treated samples than 1% BA treated and at the end of the study counts in 0.5% BA treated samples rose to 1.3E+04 CFU/ml versus 2.0E+03 CFU/ml in 1% BA treated samples, suggesting that the antifungal activity of the *B. amyloliquifaciens* spray dried fermentate is dose dependent.

### Example 10

The *B. amyloliquifaciens* strain 723 fermentate spray dried product was tested at 1% and 0.5% in preservative-free vegetable broth with a pH of 6.7 and compared to a Control and 1% organic acid-based preservative, (OA). The vegetable broth was challenged with a cocktail of two yeasts, *Saccharomyces cerevisiae,* Y-1545, and *Torulaspora delbrueckii,* Y-866, at a combined dose of approximately 300 CFU/ml. The vegetable broth was held at refrigerated temperature and analyzed over time for enumeration of yeast. Usage rates were in the range of typical inclusion in finished foods.

Referring to Figure 6, yeast counts remained at or below inoculation levels through day 40 in samples treated with 1% or 0.5% BA. Comparatively yeast counts rose steadily in both control samples and samples treated with 1% organic acid-based preservative by day 40 (2.7E+05 CFU/ml and 8.1E+05 CFU/ml, respectively). By day 104, yeast counts in 0.5% BA treated samples increased to 6.7E+05 whereas the yeast levels remained below inoculation levels in 1% BA treated samples through the duration of the study. This suggests that the antifungal activity of the *B*. *amyloliquifaciens* spray dried fermentate is dose dependent.

Accordingly, the present disclosure demonstrates effective yeast and mold control at a broad range of pH, including pH ranges where organic acid preservatives are ineffective. In addition, *Bacillus amyloliquefaciens* strain 723 fermentate exhibits a broad spectrum of inhibitory activity against both bacterial and fungal species that represent common problems for extending item shelf life in the food industry.

In the present description, certain terms have been used for brevity, clarity, and understanding. No unnecessary limitations are to be inferred therefrom beyond the requirement of the prior art because such terms are used for descriptive purposes and are intended to be broadly construed. The different apparatuses, systems, and method steps described herein may be used alone or in combination with other apparatuses, systems, and methods. It is to be expected that various equivalents, alternatives and modifications are possible within the scope of the appended claims.

This written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. A method for producing a fermentate comprising:
(a) obtaining a substrate;
(b) mixing the substrate with a strain of *Bacillus* selected from a group consisting of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis, Bacillus toyonensis,* and a GRAS species of *Bacillus* to produce a composition; and
(c) incubating the composition at a controlled temperature in a high oxygen environment to produce a fermentate.

2. The method of claim 1, wherein the strain of *Bacillus* does not produce organic acids at a level effective to inhibit the growth of microorganisms; and/or
wherein the fermentate exhibits antibacterial and antifungal activity, in particular wherein the fermentate exhibits antibacterial and antifungal activity at a pH above 5.5.

3. The method of claim 1 or 2, wherein the strain of *Bacillus* is selected from a group consisting of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis,* and *Bacillus toyonensis;* in particular wherein the strain of *Bacillus* is a *Bacillus amyloliquefaciens* strain, preferably *Bacillus amyloliquefaciens* strain 723.

4. The method according to any of the preceding claims, wherein the composition is a liquid composition and the method further comprises a step of evaporating the fermentate to produce a second fermentate; in particular wherein the method further comprises a step of spray-drying the second fermentate to produce a powdered fermentate.

5. The method according to any of the preceding claims, wherein the controlled temperature is between about 10°C and about 50°C; and/or
wherein the high oxygen environment is a liquid environment where the dissolved oxygen concentration in liquid is between 40% and 100%; in particular wherein the dissolved oxygen concentration in liquid is between 50% and 99%, and the liquid is aerated consistently by filtered atmospheric air and mechanical agitation between 50-500 RPM during incubation.

6. A method for killing or inhibiting the growth of a contaminating microorganism on or within a food product, the food product having a volume, and the method comprising:
making or obtaining a fermentate comprising a cellular mass component from a fermenting microorganism, and a substrate; and
applying an effective amount of the fermentate to the food product so as to kill or inhibit the growth of the contaminating microorganism on or within the food product;
wherein the fermenting organism is a strain of *Bacillus* selected from a group consisting of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis, Bacillus toyonensis* and a GRAS species of *Bacillus.*

7. The method of claim 6, wherein the fermentate is applied in a concentration between about 0.1% and about 5% of the food product volume.

8. The method of claim 6 or 7, wherein the strain *of Bacillus* is selected from a group consisting of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis,* and *Bacillus toyonensis;* in particular wherein the strain of *Bacillus* is a strain of *Bacillus amyloliquefaciens,* preferably *Bacillus amyloliquefaciens* strain 723.

9. The method according to any of claims 6 to 8, wherein the substrate provides a carbon source for the fermenting organism, particularly wherein the substrate is selected from the group consisting of: sucrose, dextrose, lactose, starches, fruits, vegetables, whole grains, whey, milk or extracts thereof.

10. The method according to any of claims 6 to 9, wherein the fermentate is a concentrated liquid; and/or wherein the fermentate is a dry powder.

11. The method according to any of claims 6 to 10, wherein the fermentate is produced according to any of claims 1 to 4.

12. A fermentate produced by any one of the methods of claims 1-11.

13. A food product comprising a fermentate having a cellular mass component from a strain of *Bacillus* selected from a group consisting of *Bacillus amyloliquefaciens, Bacillus marisflavi, Bacillus licheniformis, Bacillus paralicheniformis, Bacillus subtilis, Bacillus megaterium, Bacillus pumilus, Bacillus safensis, Bacillus siamensis, Bacillus tequilensis, Bacillus toyonensis* and a GRAS species of *Bacillus,* and a substrate.

14. The food product of claim 13, wherein the fermentate is obtained by a method according to any of claims 1 to 11.

15. The food product of claim 13 or 14,
wherein the food product has a pH above 5.5; and/or
wherein the food product has a volume and includes the fermentate in a concentration between about 0.1% and about 5% of the food product volume; and/or
wherein the fermentate has the ability to inhibit the growth of a contaminating microorganism up to 100% when diluted to less than 5% (w/v).
